# EUROPÄISCHE PATENTSCHRIFT

(11) **EP 2 414 046 B1**
(45) Veröffentlichungstag und Bekanntmachung des Hinweises auf die Patenterteilung: **15.03.2017**
(21) Anmeldenummer: 10709842.8
(22) Anmeldetag: 22.03.2010
(51) Int. Cl.: A61Q 5/06, A61K 8/81

(54) **MITTEL FÜR KERATINHALTIGE FASERN, ENTHALTEND MINDESTENS EIN SPEZIELLES VERNETZTES AMPHIPHILES, ANIONISCHES POLYMER UND MINDESTENS EIN WEITERES SPEZIELLES UNVERNETZTES AMPHIPHILES, ANIONISCHES POLYMER**
AGENTS FOR FIBERS CONTAINING KERATIN, CONTAINING AT LEAST ONE SPECIAL CROSS-LINKED AMPHIPHILIC, ANIONIC POLYMER AND AT LEAST ONE FURTHER SPECIAL NON-CROSS-LINKED AMPHIPHILIC ANIONIC POLYMER
AGENTS DE TRAITEMENT DE FIBRES À TENEUR EN KÉRATINE, CONTENANT AU MOINS UN POLYMÈRE ANIONIQUE AMPHIPHILE RÉTICULÉ SPÉCIAL ET AU MOINS UN AUTRE POLYMÈRE ANIONIQUE AMPHIPHILE NON RÉTICULÉ SPÉCIAL

(30) Priorität: 30.03.2009 DE 102009001978
(43) Veröffentlichungstag der Anmeldung: 08.02.2012
(73) Patentinhaber: Henkel AG & Co. KGaA, 40589 Düsseldorf (DE)
(72) Erfinder: KNAPPE, Thorsten, 22869 Schenefeld (DE); SCHEFFLER, Rene, 25479 Ellerau (DE)
(86) Internationale Anmeldenummer: PCT/EP2010/053699
(87) Internationale Veröffentlichungsnummer: WO 2010/112361

(56) Entgegenhaltungen:
- EP-A1- 1 997 472
- WO-A2-2008/098717
- US-A1- 2006 134 049
- REICHL COLLIN, JENNIFER, ET AL.: "Aculyn TM 88 rheology modifier", RESEARCH DISCLOSURE, Bd. 494, Nr. 011, Juni 2005 (2005-06), Seite 12PP, XP002661603,
- "Aculyn 88 -more than just a thickener!", ROHM AND HAAS PERSONAL CARE,, 1. April 2005 (2005-04-01), Seiten 1-2, XP007909944,

## Beschreibung

Die vorliegende Erfindung betrifft Mittel zur Haarbehandlung, enthaltend eine Kombination mindestens eines speziellen vernetzten amphiphilen, anionischen Polymers mit mindestens einem weiteren speziellen unvernetzten amphiphilen, anionischen Polymer, die Verwendung dieser Mittel zur temporären Verformung und/oder zur Pflege keratinhaltiger Fasern und Haargele auf Basis dieser Mittel. Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Wenn ein Stylingmittel als Gel zur Anwendung kommt, hat es sich als Vorteilhaft erwiesen, diese als klare, transparente Gele bereitzustellen. Diese klaren Gele empfindet der Verbraucher als ästhetisch ansprechend, insbesondere dann, wenn zusätzlich Gasblasen eingearbeitet sind. Leider lassen sich in herkömmliche Gelformulierungen oft Gasblasen schwer einarbeiten. Gelang es, Gasblasen in die herkömmlichen Rezepturen einzuarbeiten, waren die Gasblasen nicht lagerstabil eingearbeitet und entwichen aus dem Gel.

Stylingmittel zu entwickeln, die alle gewünschten Eigenschaften in Kombination aufweisen, bereitet nach wie vor Schwierigkeiten. Insbesondere gilt dies für die Kombination von ästhetischen Gesichtpunkten einerseits und starkem und flexiblem Halt andererseits. Um einen starken Halt zu vermitteln muss das fixierend wirkende Polymer gut auf der keratinhaltigen Faser haften und einen hinreichend harten Film bilden. Dennoch der resultierende Polymerfilm dem Faserkollektiv keine Taktilität eines Bretts verleihen, sondern den Fasern einen Grad an Flexibilität ermöglichen, ohne dass die aufgeprägte Formgebung des Faserkollektivs, d.h. z.B. eine Frisur, verloren geht. Aufgabe der vorliegenden Erfindung war es daher, ein Mittel zur temporären Verformung keratinischer Fasern zur Verfügung zu stellen, das sich durch einen hohen Haltegrad auszeichnet und keine Filmplaken bildet. Ferner soll das Mittel eine gesteigerte Salzverträglichkeit besitzen und eine verbesserte weiche Sensorik (Cremigkeit) ohne den Zusatz von Fettstoffen aufweisen.

Es wurde nunmehr überraschenderweise gefunden, dass dies durch die erfindungsgemäße Polymerkombination (*vide infra*) erreicht werden kann.

Ein erster Gegenstand der vorliegenden Erfindung sind daher Mittel zur Behandlung keratinhaltiger Fasern, insbesondere menschlichem Haar, enthaltend in einem kosmetisch akzeptablen Träger
(a) mindestens ein vernetztes, amphiphiles, anionisches Polymer, umfassend mindestens eine Struktureinheit der Formel (I) und mindestens eine Struktureinheit der Formel (II), 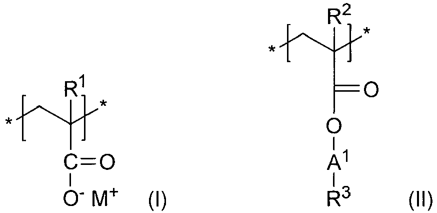 worin
   R¹ und R² stehen unabhängig voneinander für ein Wasserstoffatom oder eine Methylgruppe,
   R³ steht für eine (C₈ bis C₃₀)-Alkylgruppe,
   M⁺ steht für ein physiologisch verträgliches Kation und
   A¹ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe *-(CH₂CH₂O)x-(CH₂CHMeO)_{y}-* worin die Summe x + y für eine ganze Zahl von 5 bis 35 steht und x und y größer als Null sind
   und
(b) mindestens ein unvernetztes, amphiphiles, anionisches Polymer, umfassend mindestens eine Struktureinheit der Formel (I) und mindestens eine Struktureinheit der Formel (IV-a) und mindestens eine Struktureinheit der Formel (V), 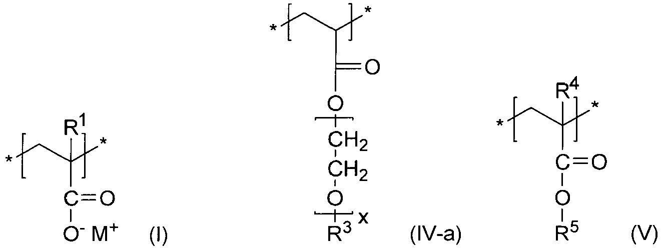 worin
   R¹ und R⁴ stehen unabhängig voneinander für ein Wasserstoffatom oder eine Methylgruppe,
   R³ steht für Palmfettalkohol,
   R⁵ steht für eine (C₂ bis C₄)-Alkylgruppe, insbesondere für Ethyl,
   M⁺ steht für ein physiologisch verträgliches Kation und
   x steht für 25.

Haarkosmetische Mittel, welche neben dem vernetzten, amphiphilen, anionischen Polymer Aculyn™ 88 (INCI: Acrylates/Steareth-20 Methacrylate Crosspolymer) weiterhin auch das unvernetzte, amphiphile, anionische Polymer Aculyn™ 28 (INCI: Acrylates/Beheneth-25 Methacrylate) enthalten, werden in der Research Disclosure "AculynTM 88 rheology modifier" (ISSN 0374-4353) beschrieben.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.

Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Gemäß obiger Formeln und allen folgenden Formeln steht eine chemische Bindung, die mit dem Symbol * gekennzeichnet ist, für eine freie Valenz des entsprechenden Strukturfragments.

Insbesondere sind als physiologisch verträgliche Kationen M⁺ zur Kompensation der negativen Ladung der amphiphilen, anionischen Polymere Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom geeignet. Letztere werden beispielsweise durch Protonierung primärer, sekundärer oder tertiärer organischer Amine mit einer Säure, oder durch permanente Quaternisierung besagter organischer Amine gebildet. Beispiele dieser kationischen organischen Ammoniumverbindungen sind 2-Ammonioethanol und 2-Trimethylammonioethanol.

Unter "vernetzt" bzw. "Vernetzung" ist im Sinne der Erfindung die Verknüpfung von Polymerketten miteinander durch kovalente chemische Bindung unter Bildung eines Netzwerkes zu verstehen. Diese kovalente Verknüpfung der Polymerketten darf mittels direkter kovalenter Bindung erfolgen oder durch ein die Polymerketten verbrückendes Molekülfragment vermittelt werden. Das Molekülfragment bindet an die durch das Molekülfragment verbrückten Polymerketten jeweils mittels kovalenter chemischer Bindung. Unter "unvernetzt" ist im Sinne der Erfindung zu verstehen, dass keine zuvor definierte "Vernetzung" vorliegt.

Unter "amphiphil" versteht der Fachmann im Allgemeinen die Tatsache, dass ein und dasselbe Molekül hydrophile Strukturelemente (beispielsweise solche der Formeln I bzw. (III)) und lipophile Strukturelemente (beispielsweise solche der Formeln (II) bzw. (IV)) umfasst.

Gemäß Formel (II) steht A¹ bevorzugt für eine Gruppe *-(CH₂CH₂O)ₓ-* worin x für eine ganze Zahl von 5 bis 35, insbesondere für eine ganze Zahl von 10 bis 24, steht.

Gemäß Formel (II) steht R² bevorzugt für eine Methylgruppe.

Die Vernetzung der vernetzen, amphiphilen, anionischen Polymere (a) kann bevorzugt durch Verwendung mindestens eines vernetzenden Monomers bewerkstelligt werden. Dabei ist es wiederum bevorzugt die vernetzenden Monomere aus mindestens einer Verbindung der Gruppe zu wählen, die gebildet wird aus polyungesättigten aromatischen Monomeren (wie beispielsweise Divinylbenzol, Divinylnaphthalin, Trivinylbenzol), polyungesättigten alicyclischen Monomeren (wie beispielsweise 1,2,4-Trivinylcyclohexan), di-funktionellen Estern der Phthalsäure (wie beispielsweise Diallylphthalat), polyungesättigte aliphatische Monomere (wie beispielsweise Diene, Triene,Tetraene wie Isopren, 1,3-Butadien, 1,5-Hexadien, 1,5,9-Decatrien, 1,9-Decadien, 1,5-Heptadien), Polyalkenylether (wie beispielsweise Triallylpentaerythritol, Diallylpentaerythritol, Diallylsucrose, Octaallylsucrose, Trimethylolpropandiallylether), polyungesättigte Ester von Polyalkoholen oder Polysäuren (wie beispielsweise 1,6-Hexandioldi(meth)acrylat, Tetramethylentri(meth)acrylat, Allylacrylat, Diallylitaconat, Diallylfumarat, Diallylmaleat, Trimethylolpropantri(meth)acrylat, Trimethylolpropandi(meth)acrylat, Polyethyleneglycoldi(meth)acrylat), Alkylenebisacrylamide (wie beispielsweise Methylenbisacrylamid, Propylenbisacrylamid) Hydroxy- und Carboxyderivate des Methylenbisacrylamids (wie beispielsweise N,N'-Bismethylolmethylenbisacrylamid), Polyethyleneglycoldi(meth)acrylate (wie beispielsweise Ethyleneglycoldi(meth)acrylat, Diethyleneglycoldi(meth)acrylat, Triethyleneglycoldi(meth)acrylat), polyungesättigte Silane (wie beispielsweise Dimethyldivinylsilan, Methyltrivinylsilan, Allyldimethylvinylsilan, Diallyldimethylsilan, Tetravinylsilan), N-Methylolacrylamid; N-alkoxy(meth)acrylamid, wobei die Alkoxygruppe eine (C₁ bis C₁₈)-Alkoxygruppe ist, ungesättigte hydrolysierbare Silane (wie beispielsweise Triethoxyvinylsilan, Trisisopropoxyvinylsilan, 3-Triethoxysilylpropylmethacrylat), hydrolyierbare Silane (wie beispielsweise Ethyltriethoxysilan, Ethyltrimethoxysilan), Epoxy-substituierte hydrolysierbare Silane (wie beispielsweise 2-(3,4-Epoxycyclohexyl)ethyltriethoxysilan, 3-Glycidoxypropyltrimethyoxysilan) Polyisocyanate (wie beispielsweise 1,4-Diisocyanatobutan, 1,6-Düsocyanatohexan, 1,4-Phenylenedüsocyanat, 4,4'-Oxybis(phenylisocyanat), ungesättigte Epoxide (wie beispielsweise Glycidylmethacrylate, Allylglycidylether), Polyepoxide (wie beispielsweise Diglycidylether, 1,2,5,6-Diepoxyhexan, Ethylenglycoldiglycidylether), ethoxylierte Polyole (wie beispielsweise Diole, Triole und Diphenole, jeweils ethoxyliert mit 2 bis 100 mol Ethyleneoxid pro Mol Hydroxylgruppen und terminiert mit einer polymerisierbaren ungesättigten Gruppe, wie beispielsweise Vinylether, Allylether, Acrylateester, Methacrylateester; Beispiele umfassen Bisphenol A ethoxyliertes di(meth)acrylat, Bisphenol F ethoxyliertes Di(meth)acrylat, ethoxyliertes Trimethylolpropantri(meth)acrylate, Acrylat- und Methacrylatester von Polyolen mit mindestens zwei Acrylatester- oder Methacrylatester-Funktionalitäten (wie beispielsweise Trimethylolpropantriacrylat (TMPTA), Trimethylolpropanethoxylated (15) triacrylat (TMPEO15TA), Trimethylolpropandimethacrylat, Triethyleneglycoldimethacrylat (TEGDMA), mit 30 Mol Ethylenoxid ethoxylliertes Bisphenol A-dimethacrylat (EOBDMA)).

Die vernetzten, amphiphilen, anionischen Polymere (a) sind in den erfindungsgemäßen Mitteln bevorzugt in einer Menge von 0,1 Gew.-% bis 5,0 Gew.-%, besonders bevorzugt von 0,2 Gew.-% bis 2 Gew.-%, ganz besonders bevorzugt von 0,2 Gew.-% bis 1,5 Gew.-% - jeweils bezogen auf das Gewicht des gesamten Mittels - enthalten.

Besonders bevorzugte erfindungsgemäße vernetzte amphiphile anionische Polymere (a) enthalten zusätzlich mindestens eine Struktureinheit der Formel (V) 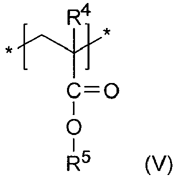 worin
R⁴ steht für ein Wasserstoffatom oder eine Methylgruppe,
R⁵ steht für eine (C₂ bis C₄)-Alkylgruppe, insbesondere für Ethyl.

Es ist erfindungsgemäß bevorzugt, als vernetztes amphiphiles, anionisches Polymer (a) mindestens ein Polymer auszuwählen, umfassend mindestens eine Struktureinheit der Formel (I) und mindestens eine Struktureinheit der Formel (II-a), 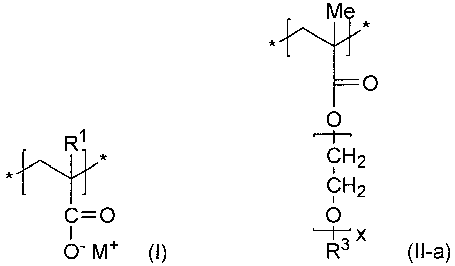 worin
R¹ steht für ein Wasserstoffatom oder eine Methylgruppe,
R³ steht für eine (C₈ bis C₃₀)-Alkylgruppe,
M⁺ steht für ein physiologisch verträgliches Kation und
x steht für eine ganze Zahl von 5 bis 35, insbesondere für eine ganze Zahl von 10 bis 24.

Darüberhinaus ist es besonders bevorzugt, die vernetzten, amphiphilen, anionischen Polymere (a) aus mindestens einem Polymer auszuwählen, umfassend mindestens eine Struktureinheit der Formel (I) und mindestens eine Struktureinheit der Formel (II-a) und mindestens eine Struktureinheit der Formel (V), 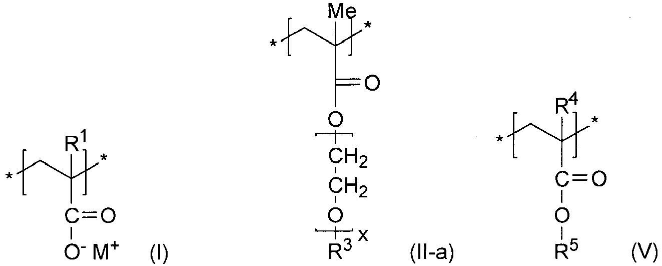 worin
R¹ und R⁴ stehen unabhängig voneinander für ein Wasserstoffatom oder eine Methylgruppe,
R³ steht für eine (C₈ bis C₃₀)-Alkylgruppe,
R⁵ steht für eine (C₂ bis C₄)-Alkylgruppe, insbesondere für Ethyl,
M⁺ steht für ein physiologisch verträgliches Kation und
x steht für eine ganze Zahl von 5 bis 35, insbesondere für eine ganze Zahl von 10 bis 24.

Ein ganz besonders bevorzugtes Polymer (a) ist ein vernetztes, amphiphiles, anionisches Polymer, das unter die INCI-Bezeichnung Acrylates / Steareth-20 Methacrylate Crosspolymer fällt. Es besitzt 20 Einheiten Ethylenoxid (x gemäß Formel (II-a) = 20) und ist mit Stearylalkohol verethert (R³ gemäß Formel (II-a) = Stearyl) Solche Polymere werden beispielsweise mit dem Handelsnamen Aculyn^{®} 88 von der Firma Rohm & Haas in Form einer 28 bis 30 Gew.-%-igen Dispersion in Wasser vertrieben.

Weiterhin enthält das erfindungsgemäße Mittel zu den zuvor definierten vernetzten amphiphilen, anionischen Polymeren zwingend mindestens ein zuvor definiertes unvernetztes, amphiphiles, anionisches Polymer (b).

Im Sinne der Erfindung bevorzugte Mittel enthalten die unvernetzten, amphiphilen, anionischen Polymere (b) in einer Menge von 0,1 Gew.-% bis 5,0 Gew.-%, besonders bevorzugt von 0,2 Gew.-% bis 5,0 Gew.-%, ganz besonders bevorzugt von 0,1 bis 2,0 Gew.%, jeweils bezogen auf das Gewicht des Mittels.

Die vernetzten, amphiphilen, anionischen Polymere (a) und die unvernetzen, amphiphilen, anionischen Polymere (b) werden erfindungsgemäß bevorzugt in einem Gewichtsverhältnis [Polymer (a) zu Polymer (b)] von 1 zu 5 bis 5 zu 1, insbesondere von 1 zu 2 bis 5 zu 1, ganz besonders bevorzugt von 1 zu 1.5 bis 2 zu 1, eingesetzt.

Das unvernetzte, amphiphile, anionische Polymer (b) besitzt 25 Einheiten Ethylenoxid (x in Formel (IV-a) = 25), ist mit Palmfettalkohol verethert (R³ in Formel (IV-a) = Alkylkettenverteilung der Palmölfettsäuren) und wird nach der INCI-Nomenklatur als Acrylates/Palmeth-25 Acrylate Copolymer bezeichnet. Ganz besonders bevorzugt enthalten daher die erfindungsgemäßen Mittel mindestens ein unvernetztes, amphiphiles, anionisches Polymer, das unter die INCI-Bezeichnung Acrylates / Palmeth-25 Acrylate Copolymer fällt. Ein solches Polymer ist beispielsweise unter der Handelsbezeichnung Synthalen^{®} W 2000 als 30 bis 32 Gew.-% ige Emulsion in Wasser von der Firma 3 V Sigma erhältlich.

Die auf den Seiten 9 bis 17 der internationalen Veröffentlichung WO 2010/112361 A2 explizit genannten Ausführungsformen (A) bis (I) stellen ganz besonders bevorzugte Ausführungsformen des erfindungsgemäßen Mittels dar. Die auf den Seiten 17 bis 19 der internationalen Veröffentlichung WO 2010/112361 A2 genannten in diesen Kombinationen (A) bis (I) bevorzugten Ausführungsformen sind auch im Rahmen dieser Anmeldung bevorzugt.

Das erfindungsgemäße Mittel enthält in einer bevorzugten Ausführungsform zusätzlich zum vernetzen, amphiphilen, anionischen Polymer (a) und dem unvernetzten, amphiphilen, anionischen Polymer (b) weiterhin zusätzlich mindestens ein filmbildendes und/oder festigendes Polymer (c). Letzteres ist von den besagten Polymeren (a) und (b) verschieden.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curlretention - Test angewendet. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch.

Das erfindungsgemäße Mittel enthält bevorzugt mindestens ein filmbildendes und/oder festigendes Polymer, das aus mindestens einem Polymer der Gruppe ausgewählt wird, die gebildet wird aus nichtionischen Polymeren, kationischen Polymeren, amphoteren Polymeren, zwitterionischen Polymeren und anionischen Polymeren, bevorzugt aus nichtionischen, kationischen und amphoteren Polymeren.

Die zusätzlichen filmbildenden und/oder festigenden Polymere sind in dem erfindungsgemäßen Mittel bevorzugt in einer Menge von 0,01 Gew.-% bis 10 Gew.-%, insbesondere von 0,5 Gew.-% bis 8,0 Gew.-%, ganz besonders bevorzugt von 0,5 Gew.-% bis 5,0 Gew.-%, jeweils bezogen auf das Gewicht des Mittels, enthalten. Diese Mengenangaben gelten auch für alle in den erfindungsgemäßen Mitteln einsetzbaren nachfolgenden bevorzugten Typen der filmbildenden und/oder festigenden Polymere. Falls nachfolgend abweichende bevorzugte Mengen spezifiziert wurden, gelten letztere als wiederum noch bevorzugter Mengen.

Besonders bevorzugt eignen sich erfindungsgemäß solche Mittel, die neben den zuvor definierten Polymeren (a) und (b) zusätzlich mindestens ein filmbildendes und/oder festigende Polymer enthalten, welches aus mindestens einem Polymer der Gruppe ausgewählt wird, die gebildet wird aus
- nichtionischen Polymeren auf Basis ethylenisch ungesättigter Monomere, insbesondere aus
   - Homopolymeren des N-Vinylpyrrolidons,
   - nichtionischen Copolymeren des N-Vinylpyrrolidons,
   - Homopolymeren und nichtionischen Copolymeren des N-Vinylcaprolactams,
   - Copolymeren des (Meth)acrylamids,
   - Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 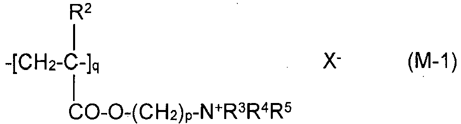 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X- ein physiologisch verträgliches organisches oder anorganisches Anion ist.

Bevorzugt als zusätzliches filmbildendes und/oder festigendes Polymer geeignete nichtionische Polymere auf Basis ethylenisch ungesättigter Monomere, sind solche nichtionischen Polymere, welche mindestens eine der nachfolgenden Struktureinheiten enthalten 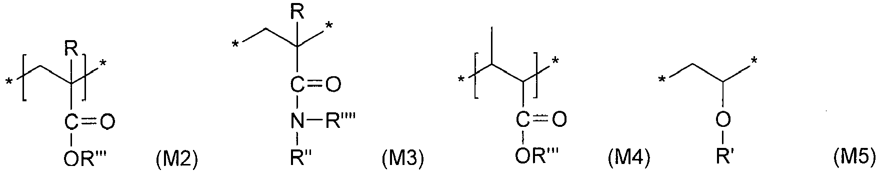 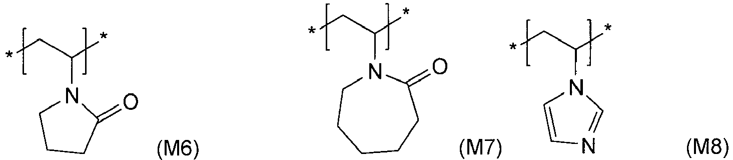 worin
- R: steht für ein Wasserstoffatom oder eine Methylgruppe,
- R': steht für ein Wasserstoffatom oder eine (C₁ bis C₄)-Acylgruppe,
- R" und R"": stehen unabhängig voneinander für eine (C₁ bis C₇)-Alkylgruppe oder ein Wasserstoffatom
- R"': steht für eine lineare oder verzweigte (C₁ bis C₄)-Alkylgruppe oder eine (C₂ bis C₄)-Hydroxyalkylgruppe.

Bevorzugte, nichtionische filmbildende und/oder nichtionische haarfestigende Polymere sind Homo-oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, wobei jeweils die Alkylgruppen dieser Monomere aus (C₁ bis C₃)-Alkylgruppen ausgewählt werden.

Für die erfindungsgemäßen Mittel besonders geeignete nichtionische Polymere auf Basis ethylenisch ungesättigter Monomere enthalten mindestens eine der nachfolgenden Struktureinheiten 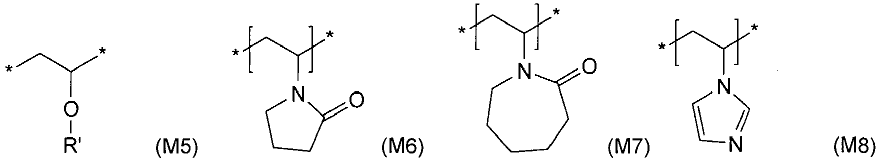 worin
- R': steht für ein Wasserstoffatom oder eine (C₁- bis C₃₀)-Acylgruppe, insbesondere für ein Wasserstoffatom oder eine Acetylgruppe.

Geeignet sind insbesondere Homopolymere des Vinylcaprolactams oder des Vinylpyrrolidons (wie beispielsweise Luviskol^{®} K 90 oder Luviskol^{®} K 85 der Firma BASF SE), Copolymerisate aus Vinylpyrrolidon und Vinylacetat (wie sie beispielsweise unter dem Warenzeichen Luviskol^{®} VA 37, Luviskol^{®} VA 55, Luviskol^{®} VA 64 und Luviskol^{®} VA 73 von der Firme BASF SE vertrieben werden), Terpolymere aus Vinylpyrrolidon, Vinylacetat und Vinylpropionat, Polyacrylamide (wie beispielsweise Akypomine^{®} P 191 von der Firma CHEM-Y), Polyvinylalkohole (die beispielsweise unter den Handelsbezeichnungen Elvanol^{®} von Du Pont oder Vinol^{®} 523/540 von der Firma Air Products vertrieben werden), Terpolymere aus Vinylpyrrolidon, Methacrylamid und Vinylimidazol (wie beispielsweise Luviset^{®} Clear der Firma BASF SE).

Neben den auf ethylenisch ungesättigten Monomeren basierenden nichtionischen Polymeren eignen sich weiterhin zur bevorzugten Ausführung der technischen Lehre nichtionische Cellulosederivate als filmbildende und/oder festigende Polymere, die bevorzugt ausgewählt werden aus Methylcellulose und insbesondere aus Celluloseether, wie Hydroxypropylcellulose (z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird), Hydroxyethylcellulose, wie sie beispielsweise unter den Warenzeichen Culminal^{®} und Benecel^{®} (AQUALON) und Natrosol^{®}-Typen (Hercules) vertrieben werden.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (M1), 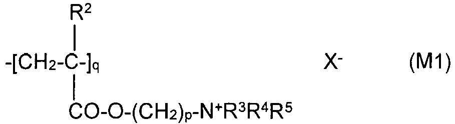 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X⁻ ein physiologisch verträgliches organisches oder anorganisches Anion ist, sowie Copolymere, bestehend im wesentlichen aus den in Formel (M1) aufgeführten Monomereinheiten sowie nichtionogenen Monomereinheiten, sind geeignete kationische filmbildende und/oder kationische festigende Polymere. Im Rahmen dieser Polymere sind diejenigen erfindungsgemäß bevorzugt, für die mindestens eine der folgenden Bedingungen gilt:
- R² steht für eine Methylgruppe
- R³, R⁴ und R⁵stehen für Methylgruppen
- m hat den Wert 2 oder 3.

Als physiologisch verträgliches Gegenionen X⁻ der Formel (M1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid. Ein gut geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere von quaternierten Derivaten des Dialkylaminoalkyl(meth)acrylats und/oder Copolymere von quaternierten Derivaten des Dialkylaminoalkyl(meth)acrylamids gelten als besonders bevorzugt geeignete kationische filmbildende und/oder kationische festigende Polymere.

Copolymere mit Monomereinheiten gemäß Formel (M1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Ein weiterhin erfindungsgemäß bevorzugt geeignetes kationisches filmbildendes und/oder kationisches festigendes Polymer ist mindestens ein kationisches Polymer, das mindestens ein Strukturelement der Formel (M9) und zusätzlich mindestens ein Strukturelement der Formel (M10) enthält 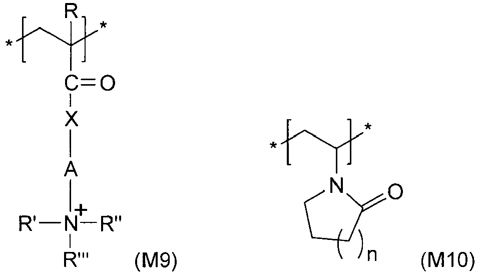 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat. Solche Verbindungen sind beispielsweise als Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN erhältlich.

Weiterhin werden die kationischen Polymere erfindungsgemäß besonders bevorzugt aus kationischen, quaternisierten Cellulose-Derivaten ausgewählt.

Ferner eignen sich als filmbildendes und/oder festigendes Polymere bevorzugt kationische, quaternisierte Cellulosederivate. Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Als im Sinne der Erfindung ganz besonders bevorzugt verwendbare kationische Polymere dienen weiterhin Copolymere, die mindestens ein Strukturelement der Formel (M11) 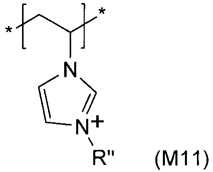 worin R" für eine (C₁ bis C₄)-Alkylgruppe, insbesondere eine Methylgruppe, steht, und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat. Es ist wiederum erfindungsgemäß bevorzugt, wenn als zusätzliches kationisches Polymer, mindestens ein Copolymer (c1) enthalten ist, das neben mindestens einem Strukturelement der Formel (M11) zusätzlich ein Strukturelement der Formel (M6) umfasst 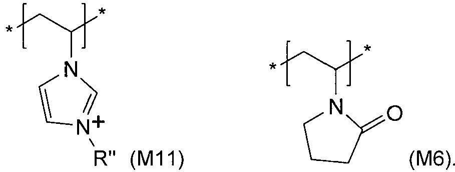 worin
R" für eine (C₁ bis C₄)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut und lassen sich durch die allgemeine Formel (Poly1) 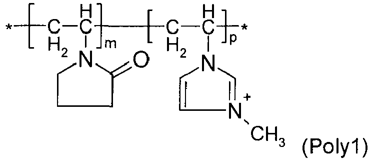 beschreiben, wobei die Indices m und p je nach Molmasse des Polymers variieren und nicht bedeuten sollen, daß es sich um Blockcopolymere handelt. Vielmehr können Struktureinheiten der Formel (M11) und der Formel (M6) im Molekül statistisch verteilt vorliegen. Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich. Besonders bevorzugte erfindungsgemäße Mittel enthalten ein Copolymer (c1), insbesondere der Formel (Poly1), das Molmassen innerhalb eines bestimmten Bereiches aufweist. Hier sind erfindungsgemäße Mittel bevorzugt, bei denen das Copolymer (c1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (M7) aufweisen

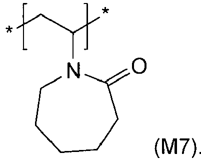

Weitere besonders bevorzugte erfindungsgemäße Mittel sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

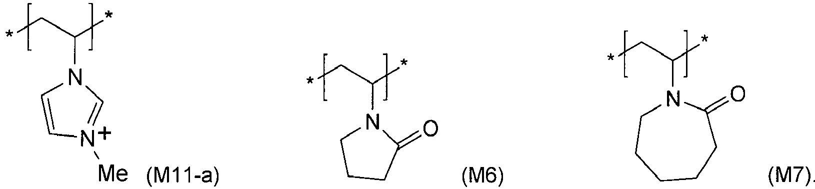

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut und lassen sich durch die allgemeine Formel (Poly2) 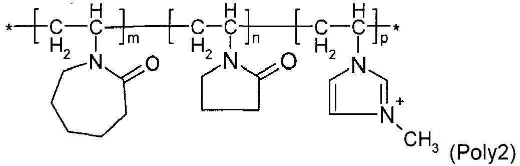 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Besonders bevorzugte erfindungsgemäße Mittel enthalten ein Copolymer (c2), das Molmassen innerhalb eines bestimmten Bereiches aufweist. Hier sind erfindungsgemäße Mittel bevorzugt, bei denen das Copolymer (c2) eine Molmasse von 100 bis 1000 kDa, vorzugsweise von 250 bis 900 kDa, weiter bevorzugt von 500 bis 850 kDa und insbesondere von 650 bis 710 kDa aufweist. Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Ganz besonders bevorzugte erfindungsgemäße Mittel sind dadurch gekennzeichnet, daß sie als zusätzliches kationisches filmbildendes und/oder kationisches festigendes Polymer mindestens ein kationisches Polymer (im folgenden Copolymer (c3)) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

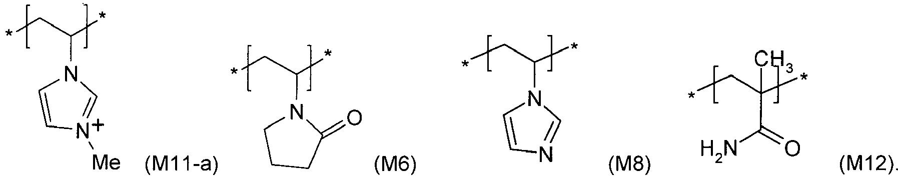

Auch hierbei ist besonders bevorzugt, wenn die Copolymere (c3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut und lassen sich durch die allgemeine Formel (Poly3) 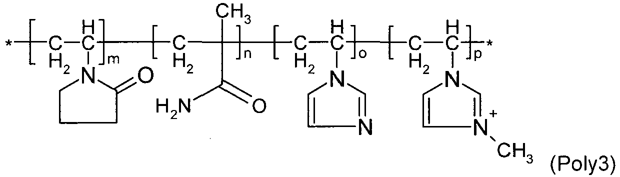 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M11-a), (M6), (M8) und (M12) im Molekül statistisch verteilt vorliegen.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Besonders bevorzugte erfindungsgemäße Mittel enthalten ein Copolymer (c3), das Molmassen innerhalb eines bestimmten Bereiches aufweist. Hier sind erfindungsgemäße Mittel bevorzugt, bei denen das Copolymer (c3) eine Molmasse von 100 bis 500 kDa, vorzugsweise von 150 bis 400 kDa, weiter bevorzugt von 250 bis 350 kDa und insbesondere von 290 bis 310 kDa aufweist. Ganz besonders bevorzugt wird das filmbildende und/oder festigende Polymer ausgewählt aus einem N-Vinylpyrrolidon/Methacrylamid/N-Vinylimidazol/1-Vinyl-3-methyl-1H-imidazolium-Quaterpolymer.

Unter den zusätzlichen filmbildenden kationischen und/oder festigenden Polymer ausgewählt aus den kationischen Polymeren mit mindesten einem Strukturelement der obigen Formel (M11-a), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren. Letztgenanntes Polymer ist ganz besonders bevorzugt. Die auf den Seiten 31 bis 43 der internationalen Veröffentlichung WO 2010/112361 A2 explizit genannten Ausführungsformen (J) bis (R) stellen ganz besonders bevorzugte Ausführungsformen des erfindungsgemäßen Mittels dar.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Zu diesen Polymeren gehören beispielsweise Chitosane. Chitosan und/oder Chitosanderivate gelten als ganz besonders bevorzugt geeignete filmbildende und/oder festigende Polymere im Sinne der vorliegenden Erfindung. Chitosane stellen Biopolymere dar und werden zur Gruppe der Hydrokolloide gezählt. Chemisch betrachtet, handelt es sich um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typischen kationischen Biopolymeren kommen im Sinne der Erfindung auch kationisch derivatisierte Chitosane (wie z. B. Quaternierungsprodukte) oder alkoxylierte Chitosane in Frage.

Erfindungsgemäß bevorzugte Mittel sind dadurch gekennzeichnet, daß sie als Chitosanderivat(e) Neutralisationsprodukte von Chitosan mit mindestens einer Säure, ausgewählt aus Milchsäure, Pyrrolidoncarbonsäure, Nicotinsäure, Hydroxyisobuttersäure, Hydroxyisovaleriansäure enthaltend oder Gemische dieser Neutralisationsprodukte umfassen.

Geeignete Chitosan(derivate) sind beispielsweise unter den Handelsbezeichnungen Hydagen^{®} CMF (1 Gew.-% Aktivsubstanz in wässriger Lösung mit 0.4 Gew.-% Glykolsäure, Molekulargewicht 500000 bis 5000000 g/mol Cognis), Hydagen^{®} HCMF (Chitosan (zu 80 % deacetyliert), Molekulargewicht 50000 bis 1000000 g/mol, Cognis), Kytamer^{®} PC (80 Gew.-% Aktivsubstanz an Chitosanpyrolidoncarboxylat (INCI-Bezeichnung: Chitosan PCA), Amerchol) und Chitolam^{®} NB/101 im Handel frei verfügbar.

Das Chitosan bzw. dessen Derivate sind in den erfindungsgemäßen Mitteln bevorzugt in einer Menge von 0,01 Gew.-% bis 20 Gew.-%, besonders bevorzugt von 0,01 Gew.-% bis 10,0 Gew.-%, ganz besonders bevorzugt von 0,1 Gew.-% bis 1 Gew.-%, jeweils bezogen auf das Gewicht des erfindungsgemäßen Mittels, enthalten.

Als im Sinne der Erfindung bevorzugt geeignete temporär kationische Polymere gelten gleichfalls solche, die mindestens eine Struktureinheit der Formeln (M1-1) bis (M1-8) aufweisen

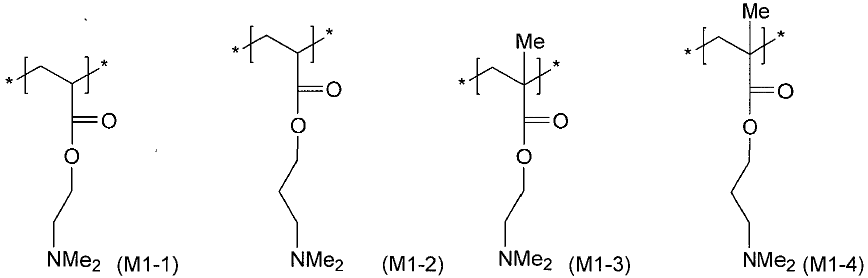

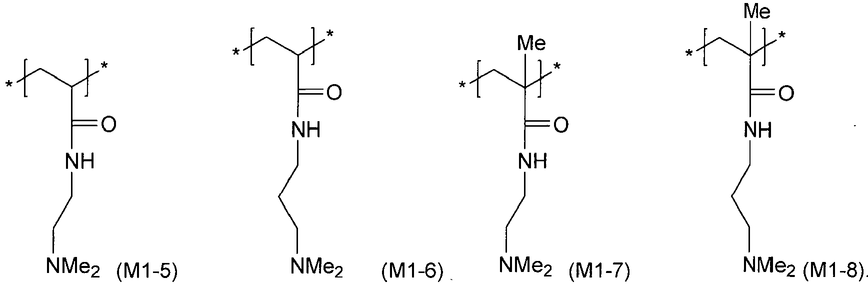

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten, 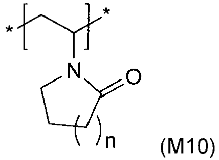 worin n 1 oder 3 bedeutet.

Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix^{®} VC 713 (ISP)),
- VinylpyrrolidonNinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex^{®} SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylamlnoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),
als bevorzugte Liste zur Auswahl.

Die erfindungsgemäßen Mittel können als filmbildendes und/oder festigendes Polymer auch mindestens ein amphoteres Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO3⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Letztere weisen zusätzlich zu der kationogenen Gruppe bzw. positiv geladenen Gruppe mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N(⁺)R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (Z-I) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (Z-II). Als Monomeres (Z-II) für die genannten Polymerisate wird bevorzugt Acrylsäure verwendet.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die amphoteren Polymere sind in den erfindungsgemäßen Mitteln bevorzugt in Mengen von 0,01 Gew.-% bis 20 Gew.-%, besonders bevorzugt von 0,05 bis 10 Gew.-%, bezogen auf das gesamte Mittel, enthalten. Mengen von 0,1 bis 5,0 Gew.-% sind ganz besonders bevorzugt.

Weiterhin können als filmbildende und/oder festigende Polymere mindestens ein anionisches filmbildendes und/oder anionisches festigendes Polymer eingesetzt werden.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere bevorzugt einsetzbare anionische Polymere werden ausgewählt aus der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INRI-Bezeichnung Polyurethane-1 von der Firma BASF SE vertrieben wird).

Falls insbesondere stark verdickend wirkende anionische Polymere zum Einsatz kommen, sollte wiederum im Rahmen einer bevorzugten Ausführungsform darauf geachtet werden, dass das zuvor genannte bevorzugte Viskositätskriterium der erfindungsgemäßen Mittel eingehalten wird. Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5,0 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- nichtionische Tenside auf Silikonbasis, insbesondere aus der Gruppe der Dimethiconcopolyole die bevorzugt alkoxyliert, insbesondere polyethoxyliert oder polypropoxyliert sind. Unter den Dimethiconcopolyolen werden als nichtionisches Tensid erfindungsgemäß besonders bevorzugt Polyoxyalkylen-modifizierte Dimethylpolysiloxane der allgemeinen Formeln (E4-V) oder (E4-VI) eingesetzt: 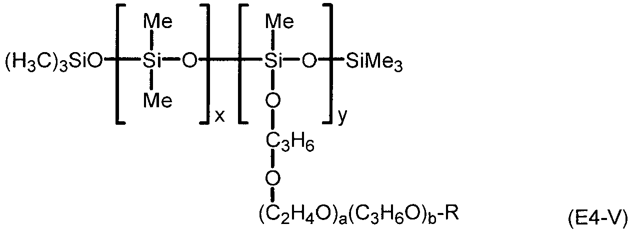 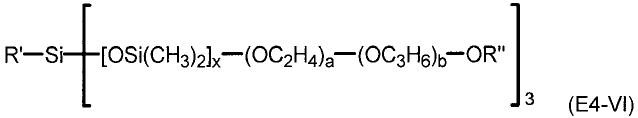 worin
   - der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
   - die Reste R' und R" bedeuten Alkylgruppen mit 1 bis 12 C-Atomen,
   - x steht für eine ganze Zahl von 1 bis 100, bevorzugt von 20 bis 30,
   - y steht für eine ganze Zahl von 1 bis 20, bevorzugt von 2 bis 10 und
   - a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.
Erfindungsgemäß besonders bevorzugte Dimethiconcopolyole sind Dow Corning 190 und Dow Corning 193 Fluid (Dow Corning).

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein.

Die erfindungsgemäßen Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Ferner enthalten die erfindungsgemäßen Mittel bevorzugt zusätzlich mindestens ein Alkanolamin. Erfindungsgemäß bevorzugte Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff wird erfindungsgemäß bevorzugt mindestens ein Silikonöl und/oder mindestens ein Silikongum eingesetzt.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole (S1) verstanden. Diese können sowohl linear als auch verzweigt als auch cyclisch oder cyclisch und verzweigt sein. Lineare Dimethiconole können durch die folgende Strukturformel (S1 - I) dargestellt werden:

(HOSiR¹₂) - O - (SiR²₂ - O - )ₓ - (Si R¹₂OH) (S1 - I)

Verzweigte Dimethiconole können durch die Strukturformel (S1 - II) dargestellt werden:

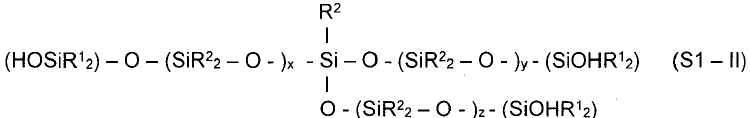

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone (S2) bilden die zweite Gruppe der Silikone, welche erfindungsgemäß enthalten sein können. Diese können sowohl linear als auch verzweigt als auch cyclisch oder cyclisch und verzweigt sein. Lineare Dimethicone können durch die folgende Strukturformel (S2 - I) dargestellt werden:

(SiR¹₃) - O - (SiR¹R²- O - )ₓ - (SiR¹₃) (S2 - I)

Verzweigte Dimethicone können durch die Strukturformel (S2 - II) dargestellt werden:

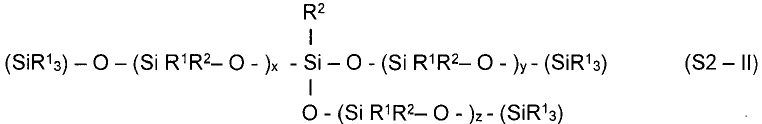

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest.

Dimethiconcopolyole (S3) bilden eine weitere Gruppe von Silikonen, die geeignet sind. Dimethiconcopolyole können durch die folgenden Strukturformeln dargestellt werden:

(SiR¹₃)-O-(SiR²2-O-)ₓ-(SiR²PE-O-)_{y}-(SiR¹₃) (S3 - I),

PE - (SiR¹₂) - O - (SiR²2 - O - )ₓ -(SiR¹₂) - PE (S3 - II)

Verzweigte Dimethiconcopolyole können durch die Strukturformel (S3 - III) dargestellt werden: 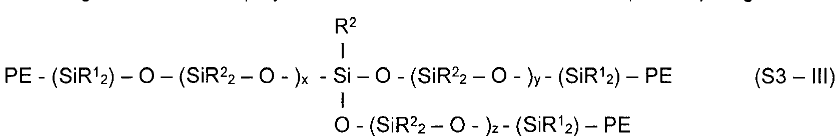 oder durch die Strukturformel (S3 - IV):

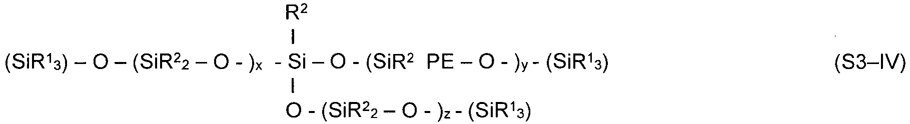

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Entsprechende Dimethiconcopolyole sind kommerziell erhältlich und werden beispielsweise von der Firma Dow Corning unter der Bezeichnung Dow Corning ^{®} 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 *m, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein. Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen.

Solche Silikone können z.B. durch die Formel (S4 - I)

M(RₐQ_{b}SiO_{(4-a-b)/2)})ₓ(R_{c}SiO_{(4-c)/2)})_{y}M (S4 - I)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel - R¹Z ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy. Z ist ein organischer, aminofunktioneller Rest, enthaltend mindestens eine funktionelle Aminogruppe. Eine mögliche Formel für Z ist NH(CH₂ )_{z}NH₂, worin z für eine ganze Zahl von 1 bis 50 steht. Eine andere mögliche Formel für Z ist -NH(CH₂)_{z}NH(CH ₂)_{zz}, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein -NHCH₂CH ₂NH₂-Rest. Eine andere mögliche Formel für Z ist - N(CH₂)_{z}NX¹X² oder - NX¹X², worin X¹ und X² jeweils unabhängig voneinander ausgewählt ist aus Wasserstoff und einem Kohlenwasserstoffrest mit 1 bis etwa 6 Kohlenstoffatomen. Ganz besonders bevorzugt steht Q für einen polaren, aminfunktionellen Rest der Formel

-CH₂CH₂CH₂NHCH₂CH₂NH ₂.

Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.

Bevorzugte aminofunktionelle Silikone entsprechen der Formel (S4 - II)

R'ₐG₃₋ₐ-Si(OSiG ₂)ₙ-(OSiG _{b}R'₂- _{b})ₘ-O-SiG₃₋ₐ-R'ₐ (S4 - II),

worin bedeutet:
- G ist -H, eine Phenylgruppe, -OH, -O-CH₃, -CH₃, -CH₂CH₃, -CH₂CH₂CH₃, -CH(CH₃)₂,-CH₂CH₂CH₂CH₃, -CH₂CH(CH₃)₂, -CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -N(R")-CH₂-CH 2- N(R")₂
   ∘ -N(R")₂
   ∘ -W(R")₃A-
   ∘ -N⁺H(R")₂ A-
   ∘ -N⁺H₂(R")A-
   ∘ -N(R")-CH₂-CH₂-N⁺R"H₂A-,
wobei jedes R" für gleiche oder verschiedene Reste aus der Gruppe -H, -Phenyl, -Benzyl, der C₁-₂₀-Alkylreste, vorzugsweise -CH₃, -CH₂CH₃, -CH₂CH₂CH₃, -CH(CH₃)₂, CH₂CH₂CH₂CH₃, -CH₂CH(CH₃)₂, -CH(CH₃)CH₂CH₃, -C(CH₃)₃, steht und A⁻ ein Anion repräsentiert, welches vorzugsweise ausgewählt ist aus Chlorid, Bromid, Iodid oder Methosulfat.

Besonders bevorzugte aminofunktionelle Silikone entsprechen der Formel (S4 - III) 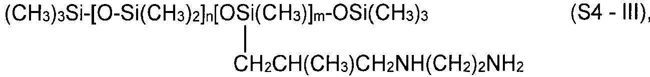 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet. Besonders bevorzugt sind weiterhin aminofunktionelle Silikone der Formel (S4 - IV) 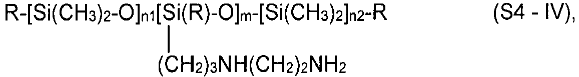 worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning^{®} 939 oder als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bzw. Silikongums bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Sericin wird beispielsweise seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" vertrieben. So wird beispielsweise unter der Handelsbezeichnung Promois^{®} Silk hydrolysiertes Fibroin mit mittleren Molekulargewichten zwischen 350 und 1000 vertrieben. Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere. Erfindungsgemäß ist es auch möglich, eine Mischung aus mehreren Proteinhydrolysaten einzusetzen. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5,0 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung. Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt. Ganz besonders bevorzugt wird als Pflegestoff D-Panthenol eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5,0 Gew.-%, jeweils bezogen auf das gesamte Mittel.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Auch Mono- bzw. Oligosaccharide können als Pflegestoff in den erfindungsgemäßen Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein. Die Verwendung wasserunlöslicher UV-Filter kann daher erfindungsgemäß bevorzugt sein.

Die UV-Filter sind üblicherweise in Mengen von 0,01-1,0 Gew.-%, bezogen auf die gesamte Anwendungszubereitung, enthalten.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitro-diphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist. Die Verbindungen Basic Yellow 87, Basic Orange 31 und Basic Red 51 sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Es ist nicht erforderlich, dass die direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Stylingergebnis nachteilig beeinflussen oder aus anderen Gründen, z.B. toxikologischen, ausgeschlossen werden müssen.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Mittel können neben den genannten Komponenten weiterhin alle für solche Zubereitungen bekannten Wirk-, Zusatz- und Hilfsstoffe enthalten. Weitere Wirk-, Hilfs- und Zusatzstoffe, sind beispielsweise Parfümöle, Lösungsmittel und -vermittler wie Ethanol, Isopropanol, Ethylenglykol, Propylenglykol, Glycerin und Diethylenglykol, quaternierte Amine wie Methyl-1-alkylamidoethyl-2-alkylimidazolinium-methosulfat, Entschäumer wie Silikone, Antischuppenwirkstoffe wie Piroctone Olamine, Zink Omadine und Climbazol, Komplexbildner wie EDTA, NTA, β-Alanindiessigsäure und Phosphonsäuren, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Konservierungsmittel, Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Vorzugsweise liegen im Rahmen einer weiteren Ausführungsform die erfindungsgemäßen Mittel in Form einer Creme oder eines Gels, insbesondere als Gel, vor. Die creme- bzw. gelförmigen erfindungsgemäßen Mittel weisen bevorzugt eine Viskosität von 10000 bis 500000 mPas, besonders bevorzugt von 30000 bis 300000 mPas, (jeweils gemessen mit Brookfield RVDV II+ mit Heilpath, Spindel T-E, 5 rpm, 20°C) auf.

Liegt das erfindungsgemäße Mittel in Form eines Gels vor, so handelt es sich besonders bevorzugt um ein transparentes Gel.

Ein zweiter Gegenstand der Erfindung ist die Verwendung der erfindungsgemäßen Mittel zur temporären Verformung von Haaren und/oder zur Haarpflege.

Die erfindungsgemäßen Mittel und Produkte, die diese Mittel enthalten, insbesondere Haargele oder Haarcremes, zeichnen sich insbesondere dadurch aus, dass sie dem behandelten Haar einen sehr starken, dauerhaften Frisurenhalt verleihen und das Haar flexibel bleibt. Wird das Mittel als Haargel konfektioniert, resultiert ein Gel mit einer teigigen Konsistenz, das sich dennoch gleichmäßig und ohne zu tropfen auf dem Haar verteilen lässt.

Es ist erfindungsgemäß bevorzugt, das Mittel des ersten Erfindungsgegenstandes als leave-on Haarbehandlungsmittel zu verwenden.

Ein dritter Gegenstand der Erfindung ist ein Verfahren zur Behandlung keratinhaltiger Fasern, insbesondere menschlicher Haare, worin das erfindungsgemäße Mittel des ersten Erfindungsgegenstandes auf die keratinhaltigen Fasern appliziert wird.

Es ist erfindungsgemäß bevorzugt, wenn die keratinhaltigen Fasern vor, während oder nach der Applikation des erfindungsgemäßen Mittels in Form gebracht werden.

Ferner gilt es erfindungsgemäß als bevorzugt, im Rahmen des erfindungsgemäßen Verfahrens das erfindungsgemäße Mittel nicht aus den keratinhaltigen Fasern auszuspülen.

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

Es wurde folgende Zusammensetzung hergestellt.

| Rohstoffe | Gew.-% |
|---|---|
| Triethanolamin | 0,79 |
| Amphomer^{® 1} | 0,80 |
| Luviquat Supreme^{® 2} | 4,50 |
| D-Panthenol | 0,15 |
| Euxyl^{®} K 320 ³ | 0,80 |
| Synthalen^{®} W2000 ⁴ | 1,70 |
| Aculyn^{®} 88 ⁵ | 2,00 |
| PEG-40 Hydrogenated Castor Oil | 0,20 |
| Uvinul^{®} P 25 ⁶ | 0,10 |
| Parfum | 0,10 |
| Wasser | ad 100 |

| | |
|---|---|
| ¹ INCI-Bezeichnung: Octylacrylamide/Acrylates/Butylaminoethyl Methacrylate Copolymer (National Starch) ² Vinylpyrrolidon-Methacrylamid-Vinylimidazol-Vinylimidazoliummethosulfat-Copolymerisat (55:29:10:6) (19-21% Festkörper in Wasser; INCI-Bezeichnung: Polyquaternium-68) (BASF SE) ³ Mischung aus Phenoxyethanol, Methylparaben, Ethylparaben und Propylenglykol (Schülke & Mayr) ⁴ INCI-Bezeichnung: Acrylates/Palmeth-25 Acrylate Copolymer (30-32 Gew.-% Polymer in Wasser) (3 V Sigma), ⁵ Copolymer aus (Meth)acrylsäure, (Meth)acrylsäureester und Steareth-20-Methacrylsäureester (28-30 Gew.-% Festkörper in Wasser; INCI-Bezeichnung: Acrylates/Steareth-20 Methacrylate Crosspolymer) (Rohm und Haas), ⁶ 1,4-Ethoxylated (25 EO) aminobenzoesäureethylester (INCI-Bezeichnung: PEG25 PABA) (BASF) | |

Das erhaltene Produkt war lagerstabil.

Bei Anwendung auf menschlichem Haar ließ sich eine hervorragende Formstabilisierung erzielen.

## Patentansprüche

1. Mittel zur Behandlung keratinhaltiger Fasern, insbesondere menschlichem Haar, enthaltend in einem kosmetisch akzeptablen Träger
(a) mindestens ein vernetztes, amphiphiles, anionisches Polymer, umfassend mindestens eine Struktureinheit der Formel (I) und mindestens eine Struktureinheit der Formel (II), 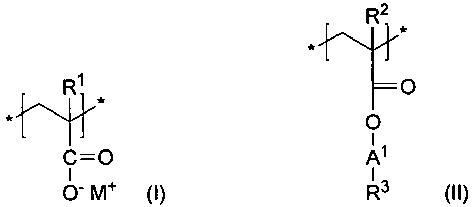 worin
R¹ und R² stehen unabhängig voneinander für ein Wasserstoffatom oder eine Methylgruppe,
R³ steht für eine (C₈ bis C₃₀)-Alkylgruppe,
M⁺ steht für ein physiologisch verträgliches Kation und
A¹ steht für eine Gruppe *-(CH₂CH₂O)ₓ-* worin x für eine ganze Zahl von 5 bis 35 steht, eine Gruppe ^{*}-(CH₂CHMeO)_{y}-* worin y für eine ganze Zahl von 5 bis 35 steht oder eine Gruppe
*-(CH₂CH₂O)ₓ-(CH₂CHMeO)_{y}-* worin die Summe x + y für eine ganze Zahl von 5 bis 35 steht und x und y größer als Null sind
und
(b) mindestens ein unvernetztes, amphiphiles, anionisches Polymer, umfassend mindestens 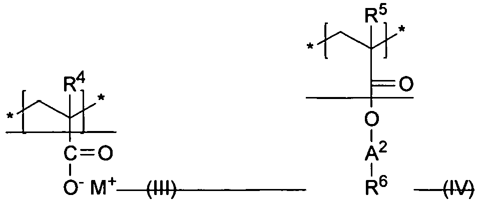 eine Struktureinheit der Formel (I) und mindestens eine Struktureinheit der Formel (IV-a) und mindestens eine Struktureinheit der Formel (V), 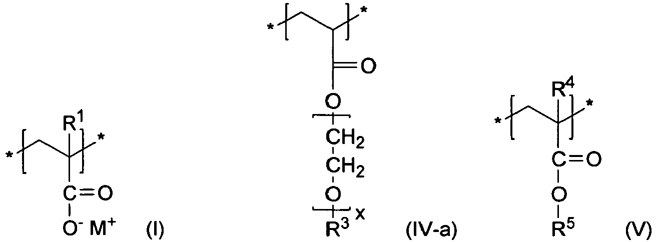 worin
R¹ und R⁴ stehen unabhängig voneinander für ein Wasserstoffatom oder eine Methylgruppe,
R³ steht für Palmfettalkohol,
R⁵ steht für eine (C₂ bis C₄)-Alkylgruppe, insbesondere für Ethyl,
M⁺ steht für ein physiologisch verträgliches Kation und
x steht für 25.

2. Mittel nach Anspruch 1, **dadurch gekennzeichnet, dass** Gemäß Formel (II) A¹ für eine Gruppe *-(CH₂CH₂O)ₓ-* steht, worin x für eine ganze Zahl von 5 bis 35, insbesondere für eine ganze Zahl von 10 bis 24, steht.

3. Mittel nach einem der Ansprüche 1 oder 2, **dadurch gekennzeichnet, dass** gemäß Formel (II) R² bevorzugt für eine Methylgruppe steht.

4. Mittel nach einem der Ansprüche 1 bis 3, **dadurch gekennzeichnet, dass** das vernetzte, amphiphile, anionische Polymer zusätzlich mindestens eine Struktureinheit der Formel (V) enthält, 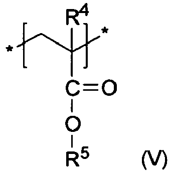 worin
R⁴ steht für ein Wasserstoffatom oder eine Methylgruppe,
R⁵ steht für eine (C₂ bis C₄)-Alkylgruppe, insbesondere für Ethyl.

5. Mittel nach einem der Ansprüche 1 bis 4, **dadurch gekennzeichnet, dass** es die vernetzten, amphiphilen, anionischen Polymere (a) in einer Menge von 0,1 Gew.% bis 5,0 Gew.-%, besonders bevorzugt von 0,2 Gew.% bis 2 Gew.%, ganz besonders bevorzugt von 0,2 Gew.% bis 1,5 Gew.%, jeweils bezogen auf das Gewicht des gesamten Mittels, enthält.

6. Mittel nach einem der Ansprüche 1 bis 5, **dadurch gekennzeichnet, dass** es die unvernetzten, amphiphilen, anionischen Polymere (b) in einer Menge von 0,1 Gew.-% bis 5,0 Gew.-%, bevorzugt von 0,2 Gew.% bis 5,0 Gew.%, besonders bevorzugt von 0,1 bis 2,0 Gew.%, jeweils bezogen auf das Gewicht des Mittels, enthält.

7. Mittel nach einem der Ansprüche 1 bis 6, **dadurch gekennzeichnet, dass** die vernetzten, amphiphilen, anionischen Polymere (a) und die unvernetzten, amphiphilen, anionischen Polymere (b) in einem Gewichtsverhältnis von 1 zu 5 bis 5 zu 1, insbesondere von 1 zu 2 bis 5 zu 1, besonders bevorzugt von 1 zu 1.5 bis 2 zu 1, eingesetzt werden.

8. Mittel nach einem der Ansprüche 1 bis 7, **dadurch gekennzeichnet, dass** zusätzlich mindestens ein filmbildendes und/oder festigendes Polymer enthalten ist.

9. Mittel nach Anspruch 8, **dadurch gekennzeichnet, dass** das filmbildende und/oder festigende Polymer ausgewählt wird aus mindestens einem kationisches Polymer, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

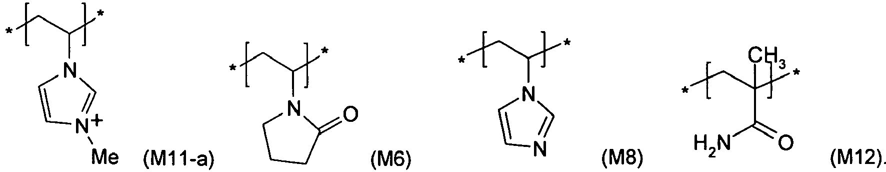

10. Mittel nach einem der Ansprüche 8 oder 9, **dadurch gekennzeichnet, dass** die filmbildenden und/oder festigenden Polymere in einer Menge von 0,01 Gew.% bis 20 Gew.%, insbesondere von 2,0 Gew.% bis 10,0 Gew.%, jeweils bezogen auf das Gewicht des Mittels, enthalten sind.

11. Mittel nach einem der Ansprüche 1 bis 10, **dadurch gekennzeichnet, dass** es zusätzlich mindestens ein Alkanolamin enthält.

12. Verwendung eines Mittels gemäß wenigstens eines der Ansprüche 1 bis 11 zur temporären Verformung von Haaren und/oder zur Haarpflege.

13. Verfahren zur Behandlung keratinhaltiger Fasern, insbesondere menschlicher Haare, worin ein Mittel gemäß wenigstens eines der Ansprüche 1 bis 11 auf die keratinhaltigen Fasern appliziert wird.

## Claims

1. An agent For treating keratin-containing fibers, in particular human hair, containing, in a cosmetically acceptable carrier:
(a) at least one crosslinked, amphiphilic, anionic polymer having at least one structural unit of formula (I) and at least one structural unit of formula (II), 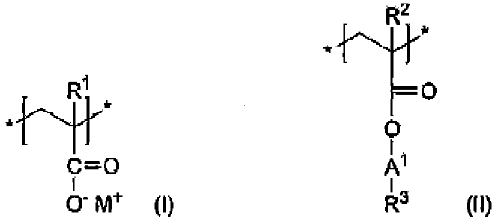 in which
R¹ and R² represent, independently of one another, a hydrogen atom or a methyl group,
R³ represents a (C₈ to C₃₀) alkyl group,
M⁺ represents a physiologically acceptable cation, and
A¹ represents a group *-(CH₂CH₂O)ₓ-* in which x represents a whole number from 5 to 35, a group *-(CH₂CHMeO)_{y}-* in which y represents a whole number from 5 to 35, or a group *-(CH₂CH₂O)ₓ-(CH₂CHMeO)_{y}-* in which the sum x + y represents a whole number from 5 to 35 and x and y are greater than zero; and
(b) at least one uncrosslinked, amphiphilic, anionic polymer containing at least one structural unit of formula (I) and at least one structural unit of formula (IV-a) and at least one structural unit of formula (V), 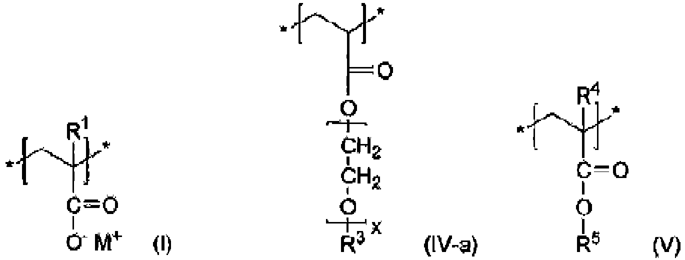 in which
R¹ and R⁴ represent, independently of one another, a hydrogen atom or a methyl group,
R³ represents palm fat alcohol,
R⁵ represents a (C₈ to C₄) alkyl group, in particular ethyl,
M⁺ represents a physiologically acceptable cation, and
x represents 25.

2. The agent according to claim 1, **characterized in that** A¹ according to formula (II) represents a group *-(CH₂CH₂Ox-* in which x represents a whole number from 5 to 35, in particular a whole number from 10 to 24.

3. The agent according to either claim 1 or claim 2, **characterized in that** R² according to formula (II) preferably represents a methyl group.

4. The agent according to one of claims 1 to 3, **characterized in that** the crosslinked, amphiphilic, anionic polymer additionally contains at least one structural unit of formula (V), 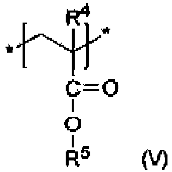 in which
R⁴ represents a hydrogen atom or a methyl group, and
R⁵ represents a (C₂ to C₄) alkyl group, in particular ethyl.

5. The agent according to one of claims 1 to 4, **characterized in that** it contains the crosslinked, amphiphilic, anionic polymers (a) in an amount of from 0.1 wt.% to 5.0 wt.%, particularly preferably of from 0.2 wt.% to 2 wt.%, very particularly preferably of from 0.2 wt.% to 1.5 wt.%, based on the weight of the total agent in each case.

6. The agent according to one of claims 1 to 5, **characterized in that** it contains the uncrosslinked, amphiphille, anionic polymers (b) in an amount of from 0.1 wt.% to 5.0 wt.%, preferably of from 0.2 wt.% to 5.0 wt.%, particularly preferably of from 0.1 to 2.0 wt.%, based on the weight of the agent in each case.

7. The agent according to one of claims 1 to 6, **characterized in that** the crosslinked, amphiphilic, anionic polymers (a) and the uncrosslinked, amphiphilic, anionic polymers (b) are used in a weight ratio of from 1 to 5 to 5 to 1, in particular of from 1 to 2 to 5 to 1, particularly preferably of from 1 to 1.5 to 2 to 1.

8. The agent according to one of claims 1 to 7, **characterized in that** at least one film-forming and/or setting polymer is additionally contained.

9. The agent according to claim 8, **characterized in that** the film-Farming and/or setting polymer is selected from at least one cationic polymer containing at least one structural unit according to formula (M11-a) and at least one structural unit according to formula (M6) and at least one structural unit according to formula (M10) and at least one structural unit according to formula (M12)

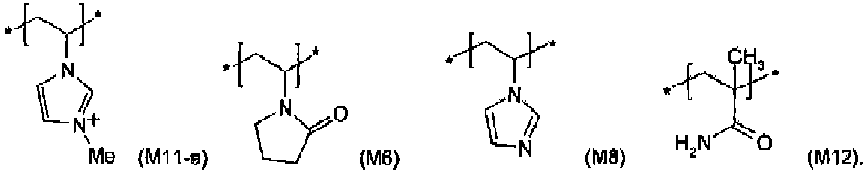

10. The agent according to either claim 8 or claim 9, **characterized in that** the film-forming and/or setting polymers are contained in an amount of from 0.01 wt.% to 20 wt.%, in particular of from 2.0 wt.% to 10.0 wt.%, based on the weight of the agent in each case.

11. The agent according to one of claims 1 to 10, **characterized in that** it additionally contains at least one alkanolamine.

12. The use of an agent according to at least one of claims 1 to 11 for temporarily shaping hair and/or for hair care.

13. A method for treating keratin-containing fibers, in particular human hair, wherein an agent according to at least one of claims 1 to 11 is applied to the keratin-containing fibers.

## Revendications

1. Produit de traitement de fibres de kératine, en particulier de cheveux humains, contenant dans un vecteur cosmétiquement acceptable:
a) au moins un polymères réticulé, amphiphile, anionique, contenant au moins une unité structurelle de formule (I) et au moins une unité structurelle de formule (II) : 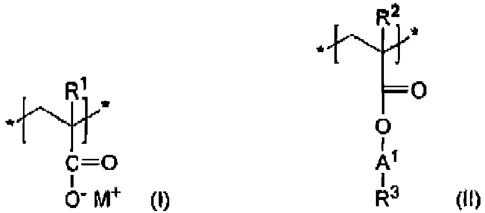 où
R¹ et R² représentent indépendamment l'un de l'autre un atome d'hydrogène ou un groupe méthyle,
R³ représente un groupe C₈₋₃₀-alkyle,
M⁺ représente un cation physiologiquement compatible, et
A¹ représente un groupe *-(CH₂CH₂O)ₓ-*, où x est un entier entre 5 et 35, un groupe *-(CH₂CHMeO)_{y}-*, où x est un entier entre 5 et 35, *-(CH₂CH₂O)ₓ-(CH₂CHMeO)_{y}-*, où la somme x+y représente un entier entre 5 et 35 et x et y sont supérieurs à 0, et
b) au moins un polymère non-réticulé, amphiphile, anionique, contenant au moins une unité structurelle de formule (I), au moins une unité structurelle de formule (IV-a) et au moins une unité structurale de formule (V) : 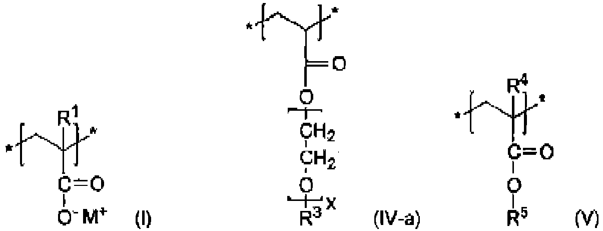 où
R¹ et R⁴ représentent indépendamment l'un de l'autre un atome d'hydrogène ou un groupe méthyle,
R³ représente l'alcool cétylique
R⁵ représente un groupe C₂₋₁-alkyle, en particulier éthyle,
M⁺ représente un cation physiologiquement compatible, et
x = 25.

2. Produit selon la revendication 1, **caractérisé en ce que** dans la formule (II), A¹ représente un groupe *-(CH₂CH₂O)ₓ-*, où x est un entier entre 5 et 35, en particulier en entier entre 10 et 24.

3. Produit selon une des revendications 1 ou 2, **caractérisé en ce que** dans la formule (II), R² représente préférentiellement un groupe méthyle.

4. Produit selon une des revendications 1 à 3, **caractérisé en ce que** le polymère réticulé, amphiphile, anionique contient en plus au moins une unité structurelle de formule (V) : 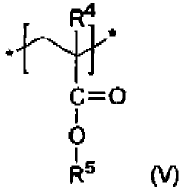 où
R⁴ représente un atome d'hydrogène ou un groupe méthyle,
R⁵ représente un groupe C₂₋₄-alkyle, en particulier méthyle.

5. Produit selon une des revendications 1 à 4, **caractérisé en ce qu'**il contient les polymères réticulés, amphiphiles, anioniques (a) à hauteur de 0,1 % à 5,0 % en poids, préférentiellement de 0,2 % à 2,0 % en poids, tout particulièrement de 0,2 % à 1,5 % en poids, rapporté respectivement au poids du produit total.

6. Produit selon une des revendications 1 à 5, **caractérisé en ce qu'**il contient les polymères non-réticulés, amphiphiles, anioniques (b) à hauteur de 0,1 % à 5,0 % en poids, préférentiellement de 0,2 % à 5,0 % en poids, tout particulièrement de 0,1 % à 2,0 % en poids, rapporté respectivement au poids du produit total.

7. Produit selon une des revendications 1 à 6, **caractérisé en ce que** les polymères réticulés, amphiphiles, anioniques (a) et les polymères non-réticulés, amphiphiles, anioniques (b) sont utilisés dans un rapport pondéral de 1:5 à 5:1, préférentiellement de 1:2 à 5:1, en particulier de 1:1,5 à 2:1.

8. Produit selon une des revendications 1 à 7, **caractérisé en ce qu'**il contient en plus au moins un polymère filmogène et/ou solidifiant.

9. Produit selon la revendication 8, **caractérisé en ce que** le polymère filmogène et/ou solidifiant est choisi parmi au moins un polymère cationique qui contient au moins une unité structurelle de formule (M11-a), au moins une unité structurelle de formule (M6), au moins une unité structurelle de formule (M10) et au moins une unité structurelle de formule (M12) :

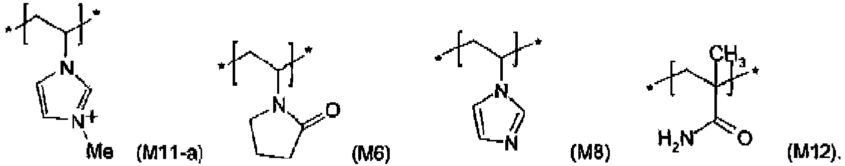

10. Produit selon la revendication 8 ou 9, **caractérisé en ce que** les polymères filmogènes et/ou solidifiants sont contenus à hauteur de 0,01 % à 20 % en poids, en particulier de 2,0 % 10,0 % en poids, rapporté au poids du produit.

11. Produit selon une des revendications 1 à 10,**caractérisé en ce qu'**il contient en plus au moins une alcanolamine.

12. Utilisation d'un produit selon au moins une des revendications 1 à 11 pour la déformation temporaire de cheveux et/ou pour les soins capillaires,

13. Procédé de traitement de fibres de kératine, en particulier de cheveux humains, où on applique un produit selon au moins une des revendications 1 à 11 sur les fibres de kératine.
